# EUROPEAN PATENT APPLICATION

(11) **EP 3 505 158 A1**
(43) Date of publication of application: **03.07.2019**
(21) Application number: 17210683.3
(22) Date of filing: 27.12.2017
(51) Int. Cl.: A61K 9/16, A61K 9/20, A61K 9/48, A61K 31/00

(54) **PHARMACEUTICAL COMPOSITION OF LENALIDOMIDE PHARMACEUTICALLY ACCEPTABLE ACID ADDITION SALT**

(71) Applicant: KRKA, d.d., Novo mesto, 8501 Novo mesto (SI)
(72) Inventor: Baselj, Nastja, 8000 Novo mesto (SI); Pisanec, Gasper, 4226 Ziri (SI); Korasa, Klemen, 1000 Ljubljana (SI); Smodis, Jani, 8323 Ursna sela (SI); Slanc Vovk, Janika, 1000 Ljubljana (SI); Kolenc, Ivanka, 8000 Novo mesto (SI)
(74) Representative: Hoffmann Eitle

(57) **Abstract**

The present invention provides solid pharmaceutical formulations and methods for making the same, wherein the pharmaceutical formulations comprise lenalidomide in the form of a pharmaceutically acceptable salt. The pharmaceutical formulations of the present invention exhibit superior stability especially in terms of disproportionation of the lenalidomide salt: this undesired effect is prevented by incorporating an acid into the formulation and/or by avoiding the use of proton-accepting excipients and/or by selecting a combination of excipients such that the resulting formulation exhibits acidic properties.

## Description

### Field of the invention

The present invention relates to a pharmaceutical composition comprising lenalidomide pharmaceutically acceptable acid addition salt, preferably hydrochloride, exhibiting superior chemical and physical storage stability, as well as to a process for the preparation thereof. Addition of an acid and selection of excipients which are not capable of accepting proton can enhance the physicochemical stability of lenalidomide in the pharmaceutical composition, in particular in the finished dosage form, and enable the required dissolution and bioavailability of the drug.

### Background of the invention

Lenalidomide is a common name for (3-(4' aminoisoindoline-1'-one)-1-piperidine-2, 6-dione), a compound of formula (I):

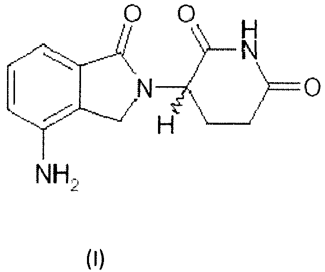

Although it is chiral and possesses an asymmetric carbon, it has been developed as a racemic mixture since it undergoes racemisation under physiological conditions.

The lenalidomide mechanism of action includes anti-neoplastic, anti-angiogenic, pro-erythropoietic, and immunomodulatory properties. Specifically, lenalidomide inhibits proliferation of certain haematopoietic tumour cells (including multiple myeloma [MM] plasma tumour cells and those with deletions of chromosome 5), enhances T cell- and Natural Killer (NK) cell-mediated immunity and increases the number of NK T cells, inhibits angiogenesis by blocking the migration and adhesion of endothelial cells and the formation of microvessels, augments foetal haemoglobin production by CD34+ haematopoietic stem cells, and inhibits production of pro-inflammatory cytokines (e.g., TNF-α and IL-6) by monocytes.

Lenalidomide is used for the treatment of adult patients with previously untreated multiple myeloma, myelodysplastic syndrome, relapsed or refractory mantle cell lymphoma.

Lenalidomide is available on the market in the form of a free base and is sold under trade name Revlimid, as an oral hard capsule in 2.5 mg, 5 mg, 7.5 mg, 10 mg, 15 mg, 20 mg and 25 mg strengths. The capsule comprises anhydrous lactose (as diluent), microcrystalline cellulose (as diluent), croscarmellose sodium (as disintegrant) and magnesium stearate (as lubricant) as inactive ingredients.
Reported dissociation constant pKa₁ for lenalidomide primary amine group is 2.31, pKa₂ for secondary amine group is 11.6.

EP 925294 discloses lenalidomide, process for production thereof, its use and pharmaceutical composition thereof. The patent discloses *inter alia* the composition of a capsule comprising active ingredient in admixture with non-toxic pharmaceutically acceptable excipients, which are microcrystalline cellulose, sodium lauryl sulfate, magnesium stearate.

WO 2005023192 is related to specific crystalline forms of lenalidomide selected from anhydrous forms, dihydrates and hemihydrates. As a most stable form hemihydrate in polymorphic form B is disclosed, this is present in the marketed form.

WO 2011050962 discloses acid addition salts of lenalidomide, process for production thereof as well as oral dosage form thereof. Disclosed are acid addition salts with acids having pKa value in water at 25°C from -10 to +4. Preferably strong acids having pKa value of less than (-2) are used. It is reported that by using strong acids dissociation is present at smaller extent.

The document discloses also polymorphic forms of acid addition salts. Lenalidomide hydrochloride of polymorphic form A is disclosed showing characteristic peaks in X-ray powder difractogram (XRPD) at 12.11, 24.35, 24.73, 26.12 ° 2- theta and lenalidomide sulfate in polymorphic form A and B are specifically disclosed. Pharmaceutical dosage form of lenalidomide acid addition salt is disclosed comprising filler, preferably a lactose-free filler, and /or solubilizer and/or disintegrant. The mean particle size of the active ingredient is from 0.1 to 100 microns, preferably from 1 to 20 microns. The document discloses pharmaceutical compositions comprising lactose and/or microcrystalline cellulose or dicalcium phosphate dihydrate as a filler, croscarmelose sodium as a disintegrant and magnesium stearate as a lubricant. These excipients are similar to those present in the marketed formulation of lenalidomid.

US 2011060010 discloses pharmaceutically acceptable strong acid salts of lenalidomide, process for preparing the same, polymorphic forms and their use as medicament. Inter alia salts with hydrochloric acid, hydrobromic acid, sulfuric acid, nitric acid, benzene sulfonic acids, p-toluene sulfonic acid, methane sulfonic acid are specifically mentioned. Further it is reported that weak acids, such as benzoic acid, succinic acid, oxalic acid, fumaric acid, maleic acid, acetic acid do not form salts with lenalidomide. The main advantage of the salts is improved water solubility which is 4 to 5000 times higher than solubility of lenalidomide. This document does not disclose any specific pharmaceutical composition.

WO 2011018101 discloses acid addition salts of lenalidomide wherein the acid has a pKa lower than 1, specifically mentioned are hydrochloric acid, hydrobromic acid, methane sulfonic acid, ethane sulfonic acid, benzene sulfonic acids and p-toluene sulfonic acid. The document discloses the use of the salts in the process of purifying lenalidomide base.

Co-crystals of lenalidomide are disclosed in WO2013012485 and WO2014160686.

WO2010054833 and WO2009114601 disclose solid dispersions containing amorphous lenalidomide. It has been found that amorphous lenalidomide generates more impurities, especially oxidative impurities, if compared with the crystalline lenalidomide, making the use of amorphous lenalidomide in a commercial scale very difficult.

WO2017032870 discloses pharmaceutical composition comprising active substance lenalidomide in the form selected from base, co-crystal, solvate or a salt wherein lenalidomid is having particle size distribution d(90) ranging from 1 to 100 microns. Exemplified pharmaceutical composition comprises lactose, microcrystalline cellulose, sodium croscarmelose and magnesium stearate, i.e. the same composition was used irrespective of which form of lenalidomide was comprised in the formulation.

As evident form the prior art in the literature there are reported several pharmaceutical compositions comprising lenalidomide salts. The disclosed compositions comprise inactive ingredients comprising lactose and compounds capable of accepting proton, such as magnesium stearate, sodium croscarmellose, dicalcium phosphate, sodium lauryl sulfate. Said prior art compositions do not exhibit satisfactory stability and, at the same time, satisfactory solubility.

Thus in view of the stability problems of lenalidomide in amorphous form or in the form of pharmaceutically acceptable salt, there is a need for pharmaceutical compositions comprising lenalidomide, which are physically and chemically stable, have improved dissolution profile and are suitable for use on a commercial scale.

### Summary of the invention

According to the present invention there is disclosed a pharmaceutical formulation, comprising (a) as the active ingredient, lenalidomide in the form of pharmaceutically acceptable acid addition salt, (b) an acid and (c) at least one further pharmaceutically acceptable excipient.

According to another embodiment of the present invention there is disclosed a pharmaceutical formulation, comprising (a) as the active ingredient, lenalidomide in the form of pharmaceutically acceptable acid addition salt, (b) optionally an acid, (c) at least one further pharmaceutically acceptable excipient and (d) wherein it is free of excipients capable of accepting proton.

According to the preferred embodiment of present invention there is disclosed a pharmaceutical formulation, comprising (a) as the active ingredient, lenalidomide in the form of pharmaceutically acceptable acid addition salt, (b) an acid, (c) at least one further pharmaceutically acceptable excipient and (d) wherein it is free of excipients capable of accepting proton.

According to a preferred embodiment of the present invention there is disclosed a pharmaceutical formulation, comprising (a) as the active ingredient, lenalidomide in the form of hydrochloric acid addition salt, (b) an acid and, (c) at least one further pharmaceutically acceptable excipient.

According to another preferred embodiment of the present invention there is disclosed a pharmaceutical formulation, comprising (a) as the active ingredient, lenalidomide in the form of hydrochloric acid addition salt, (b) optionally an acid, (c) at least one further pharmaceutically acceptable excipient and (d) wherein it is free of excipients capable of accepting proton.

According to the preferred embodiment of present invention there is disclosed a pharmaceutical formulation, comprising (a) as the active ingredient, lenalidomide in the form of hydrochloric acid addition salt, (b) an acid, (c) at least one further pharmaceutically acceptable excipient and (d) wherein it is free of excipients capable of accepting proton.

According to yet another preferred embodiment, there is disclosed a pharmaceutical formulation, comprising (a) as the active ingredient, lenalidomide in the form of hydrochloride acid addition salt, (b) one or more pharmaceutically acceptable excipients, wherein the excipients are selected such that the pharmaceutical composition shows acidic properties.

According to another embodiment there is provided a process for manufacturing the pharmaceutical formulation which comprises
- mixing lenalidomide pharmaceutically acceptable salt and at least one excipient or a mixture of excipients to form a powder mixture which can be optionally sieved before mixing to give a mixture;
- sieving the mixture, optionally addition of further excipients or mixture of excipients
- optionally blending the mixture;
- preparing the desired dosage form.

According to a preferred embodiment of the present invention, the process comprises:
- mixing lenalidomide pharmaceutically acceptable salt and a portion of diluent in a weight/weight ratio from 1:1 to 1: 50, preferably from 1:2 to 1:30, more preferably from 1:3 to 1:25, and/or further excipients and sieving the mixture
- mixing the above obtained mixture with the remaining portion of diluent and/or further excipients in a mixer to obtain homogenized mixture,
- optionally dry sieving the mixture through a sieve in order to segregate cohesive particles and to improve content uniformity,
- addition of lubricant and/or glidant and mixing the mixture to obtain a mixture,
- preparing the desired solid dosage form,

### Detailed description of the invention

### Definitions

The term "**excipient capable of accepting proton**" means an excipient, which shows moderate or strong alkaline reactivity, i.e. which has a pKb value of less than 12.This group comprises bases, salts of weak acids, wherein the acid is having pKa of more than 2, preferably more than 3 more preferably more than 4", such as salts comprising anion derived from carboxylic acid, silicic acid, phosphoric acid, carbonic acid, and salts of alcohols. Such alkaline compounds are detrimental because they can accept the proton of conjugated lenalidomide acid and thus can cause formation of lenalidomide free base.

A formulation of the present invention exhibits **acidic properties** if a suspension of the formulation in water exhibits a pH value in the range of 1 to 5, more preferably in the range of 1 to 4, most preferably 1 to 3. The pH value is determined by measuring the pH value of a 20% w/v suspension prepared by dispersing a powdered form of the pharmaceutical composition in an appropriate amount of purified water, using a pH meter such as pH Radiometer PHM240. The detailed description of pH determination of pharmaceutical formulations is further disclosed in the examples section of the present application.

The term **excipient** has its normal meaning, i.e. it refers to any pharmaceutically acceptable substance that has no therapeutic activity as such but is present in the formulation for other reasons, e.g. to improve dissolution of the active pharmaceutical ingredient (API) itself. Pharmaceutically acceptable excipients may for instance be selected from diluents, lubricants and/or glidants, disintegrants and binders.

The term "**tablet**" as used herein is intended to encompass compressed pharmaceutical dosage formulations of all shapes and sizes. Uncoated tablets are particularly preferred. The term "**capsule**" encompasses pharmaceutical dosage form formulations wherein the active pharmaceutical ingredient, optionally mixed with further excipients, is enclosed in a capsule shell.

**Low substituted hydroxypropylcellulose (L-HPC)** refers to a cellulose derivative having a degree of substitution with hydroxypropoxy-groups that does not exceed 20%, typically less than 15%, of the cellulose hydroxyl groups.

The term **average particle size** refers to the volume average or volume mean diameter of the particle size distribution. The average particle size can be determined by laser light scattering using e.g. a Malvern Mastersizer Apparatus 2000. Particle sizes are determined by measuring the angular distribution of laser light scattered by a homogeneous suspension of particles. The particles to be subjected to the particle size measurement are first suspended in appropriate non-polar dispersant such as n-hexane and then subjected to a size determination in a Malvern Mastersizer 2000 instrument. Usually, 100-800 mg of substance is dispersed in 5-10 mL of dispersant. Particle size of lenalidomide pharmaceutically acceptable salt is preferably determined by dispersion unit Hydro S. Particle size of the excipients is preferably determined by means of the Scirocco unit of the Malvern Mastersizer 2000, wherein the sample is dispersed in an air flow.

**Particle size distribution** can also be determined by laser light scattering using e.g. a Malvern Mastersizer Apparatus 2000 in the same manner as described above for the average particle size.

The values given as **d(0.1)**, **d(0.5)** and **d(0.9)** indicate that 10%, 50% and 90% of the particles, respectively, are smaller than the specified values.

Two particle distributions are considered to have the **same particle size range** if the average particle size of at least one of the two particle size distributions lies between the d(0.1) and the d(0.9) values of the other particle distribution.

**Moisture content** is specified in % by weight and it is determined by the Karl Fischer method, disclosed in Ph. Eur. version 9.2, monograph 2.5.12 (monograph number 07/2015:20512).

**Loss on drying** is specified in % by weight and is determined by weighing a sample, drying it until it reaches constant weight and weighing the sample again. Drying is preferably done by a halogen dryer, such as Mettler Toledo HR73 or R 83 or equivalent equipment. Preferred drying conditions are drying at 105°C until constant weight is reached.

**Low gas and moisture permeable primary packaging** means a packaging that has a water vapour transmission rate (WVTR value according to ASTM F 1249) of no more than 0.5 g/m²/day at 40°C and 75%RH, preferably of no more than 0.25 g/m²/day at 40°C and 75%RH and an oxygen transmission rate (OTR value according to ASTM D 3985) of no more than 2 ml/m²/day*bar at 23°C and 50%RH, preferably no more than 1 ml/m²/day*bar at 23°C and 50%RH.

Unless specified otherwise, all percentages indicated herein are % by weight based on the total weight of the pharmaceutical formulation.

### Overview

During their work the inventors have observed that the compositions comprising lenalidomide acid addition salts prepared according to the teaching of the prior art do not meet requirements for storage stable formulations. It has been observed that lenalidomide acid addition salt in formulations comprising compounds capable of accepting proton are not physically stable and that disproportionation takes place in substantial amounts, which results in partial or complete formation of free base form of lenalidomide. This is evidenced in more detail in the Reference Examples.

As disclosed in the prior art, the free base form and acid addition salt forms can have significantly different solubility, and any changes of physical form during production step and on storage can affect bioavailability of such drug forms. The main aim of the inventors was to provide physically and chemically stable form of the lenalidomide acid addition salt in the formulation.

It has been found out that desired storage stability, in-vitro dissolution and processability of lenalidomide pharmaceutical composition is highly dependent on the nature of the inactive pharmaceutical ingredients used.

Most of the pharmaceutical compositions disclosed in the prior art comprise lactose. Lactose is a reducing sugar that is capable of reacting with compounds carrying an amino group in the Maillard reaction. If a drug carries an amino group, it can degrade via the Maillard reaction. The drugs that comprise primary or secondary amines are particularly susceptible to degradation due to Maillard reaction. Lenalidomide is a primary amine. Therefore, it was the aim of inventors to develop a composition which allows to prevent degradation of lenalidomide via the Maillard reaction.

It has been unexpectedly found out that by addition of an acid and/or the use of the pharmaceutically acceptable excipients, which are free of excipients capable of accepting protons and/or the choice of excipients such that the formulation reacts acidic, target storage stability and dissolution profile similar to that obtained for marketed product Revlimid can be achieved. Surprisingly, this stabilization effect is observed even in the presence of lactose or other reducing sugars. Nonetheless, it is preferred to avoid using any reducing sugar as this may help to improve stability even further.

The present invention provides pharmaceutical formulations that allow to accomplish such advantageous stabilization effects while maintaining excellent dissolution characteristics of the API and suitability for industrial scale production. In particular, three embodiments of the pharmaceutical formulation of the present invention are provided.

According to a first embodiment of the present invention there is disclosed a pharmaceutical formulation, comprising (a) as the active ingredient, lenalidomide in the form of pharmaceutically acceptable acid addition salt, (b) an acid and (c) at least one further pharmaceutically acceptable inactive ingredient.

According to a second embodiment of the present invention there is disclosed a pharmaceutical formulation, comprising (a) as the active ingredient, lenalidomide in the form of pharmaceutically acceptable acid addition salt, (b) optionally an acid, (c) at least one further pharmaceutically acceptable inactive ingredient and (d) wherein it is free of excipients capable of accepting proton.

According to a third embodiment of the present invention, there is disclosed a pharmaceutical formulation, comprising (a) as the active ingredient, lenalidomide in the form of pharmaceutically acceptable acid addition salt, (b) one or more pharmaceutically acceptable excipients, wherein the excipients are selected such that the pharmaceutical composition shows acidic properties.

### The pharmaceutical Formulation

The following characteristics are common to the pharmaceutical formulations of all three embodiments of the invention.

The pharmaceutical formulations of the present invention are solid pharmaceutical formulations. The formulations can be in the form of capsules, powders, tablets, minitablets, microtablets, coated tablets, coated minitablets, coated microtablets, pills, lozenges, etc. The pharmaceutical formulations of the present invention are preferably suitable for oral application. The formulations of the present invention preferably have the form of capsules or tablets.

The pharmaceutical compositions of the present invention are preferably formulated in a unit dosage form, each dosage form containing about 0.1 to about 50 mg of lenalidomide in the form of pharmaceutically acceptable salt, preferably about 1 mg to about 30 mg of lenalidomide in the form of pharmaceutically acceptable salt, more preferably oral dosage form comprising 2.5 mg, 5 mg, 7.5 mg, 10 mg, 15 mg or 20 mg or 25 mg of lenalidomide in the form of pharmaceutically acceptable salt. The amounts given are calculated on the content of lenalidomide free base.

### The Active Pharmaceutical Ingredient

The following characteristics of the API apply to all three embodiments of the present invention.

The component (a), lenalidomide pharmaceutically acceptable acid addition salt, can be selected from the salts disclosed in WO2011050962, such as hydrochloride, sulfate or hydrogen sulfate, from the salts disclosed in US 2011/0060010 such as hydrochloride, hydrobromide, nitrate, besylate, tosylate, mesylate, and sulfate or the salts disclosed in WO2011018101 such as salts of lenalidomide with acids having pKa below 1, such as lenalidomide besylate and lenalidomide tosylate. Preferably, the present invention relies on the use of lenalidomide hydrochloride salt, more preferably lenalidomide hydrochloride monohydrate, most preferably lenalidomide hydrochloride monohydrate in polymorphic form VIIA, as disclosed in US2011/0060010.

According to one aspect of any one of the embodiments of the present invention, the lenalidomide pharmaceutically acceptable salt has an average particle size in the range of 1 and 150 µm, preferably between 5 and 100 µm and most preferably between 8 and 50 µm.

In another aspect of any of the embodiments of the present invention, the lenalidomide pharmaceutically acceptable salt has a particle size distribution of d(0.1) from 0.1 to 20 µm, d(0.5) from 1 to 150 µm, d(0.9) from 10 to 300 µm, preferably d(0.1) from 0.5 to 10 µm, d(0.5) from 3 to 100 µm, d(0.9) from 15 to 150 µm, more preferably d(0.1)from 0.5 to 5 µm, d(0.5) from 4 to 70 µm, d(0.9) from 20 to 100 µm.

### The acid component

The acid component (b) is an essential component of the first embodiment and it is an optional component of the second and third embodiment. The following characteristics apply to all three embodiments.

The acid component (b) is preferably a pharmaceutically acceptable acid. It can be selected from the group of acids and mixtures thereof, especially from the group of organic acids, inorganic acids, and mixtures thereof. An organic acid can be in particular selected from compounds comprising at least one (e.g. one or two or more) carboxylic acid moiety, compounds comprising at least one (e.g. one or two or more) sulfonic acid moiety, and mixtures thereof. Preferably, an organic acid has a total number of carbon atoms of at least 2, preferably at least 4, and up to 20, preferably up to 16, more preferably up to 10. Preferably acids are used that have a pKa of less than 4, more preferably less than 3. It is particularly preferred to use acids, which have a pKa of 0 or more, preferably pKa of 1 or more. Acids with multiple acidic groups have multiple pKa values. The pKa indication provided herein refers to the first deprotonation, i.e. to the most acidic proton (in other words, it is the lowest pKa). A stronger acid may also be used, but then it is advisable to select the same acid that was used for forming a salt with lenalidomide. In this manner, it is possible to minimize the risk that (part of) the lenalidomide salt is converted to a different salt form.

Organic acids can be for example selected from compounds of formula (II), compounds of formula (III), compounds of formula (IV), compounds of formula (V), and mixtures thereof:

R¹-COOH (II)

R²-SO₃H (III)

HOOC-R³-COOH (IV)

HO₃S-R⁴-SO₃H (V)

wherein R¹ can be selected from linear or branched C₁-C₂₀ alkyl (preferably linear or branched C₁-C₁₅ alkyl, especially linear or branched C₂-C₉ alkyl), optionally substituted with one or more substituents selected from -OH, -SO₃H, and C₁-C₄-alkyl; C₆-C₁₂ cycloalkyl, optionally substituted with one or more substituents selected from -OH, -SO₃H, and C₁-C₄-alkyl; linear or branched C₂-C₂₀ alkenyl, optionally substituted with one or more substituents selected from -OH, -SO₃H, and C₁-C₄-alkyl; C₆-C₁₂ aryl, optionally substituted with one or more substituents selected from -OH, -SO₃H, and C₁-C₄-alkyl;
wherein R² can be independently selected from linear or branched C₁-C₂₀ alkyl (preferably linear or branched C₁-C₁₅ alkyl, especially linear or branched C₂-C₉ alkyl), optionally substituted with one or more substituents selected from -OH, -COOH, and C₁-C₄-alkyl; C₆-C₁₂ cycloalkyl, optionally substituted with one or more substituents selected from -OH, -COOH, and C₁-C₄-alkyl; linear or branched C₂-C₂₀ alkenyl, optionally substituted with one or more substituents selected from -OH, -COOH, and C₁-C₄-alkyl; C₆-C₁₂ aryl, optionally substituted with one or more substituents selected from -OH, -COOH, and C₁-C₄-alkyl;
wherein R³ and R⁴ can be independently selected from linear or branched C₁-C₂₀ alkylene (preferably linear or branched C₁-C₆ alkylene), optionally substituted with one or more substituents selected from -OH, -COOH, -SO₃H, and C₁-C₄-alkyl; C₆-C₁₂ cycloalkylene, optionally substituted with one or more substituents selected from -OH, -COOH, -SO₃H, and C₁-C₄-alkyl; linear or branched C₂-C₂₀ alkenyl, optionally substituted with one or more substituents selected from -OH, -COOH, -SO₃H, and C₁-C₄-alkyl; and C₆-C₁₂ arylene, optionally substituted with one or more substituents selected from - OH, -COOH, -SO₃H, and C₁-C₄-alkyl. Preferably, R³ and R⁴ can be independently selected from linear or branched C₁-C₆ alkylene (especially a C₁ or C₂ or C₃ or C₄ or C₅ alkylene), which is unsubstituted or which is substituted with at least one (e.g. 1, 2, 3, 4) -OH substituent, and is optionally further substituted with at least one -COOH substituent.

Preferably, the organic acid is selected from the group consisting of oxalic, pyruvic, malonic, lactic, glyceric, fumaric, maleic, oxaloacetic, tartaric, dihydroxytartaric, oxoglutaric, citric, salicylic, gentisic, phthalic acid or a mixture thereof. More preferably, the organic acid is selected from oxalic, maleic, pyruvic, malonic, tartaric acid or a mixture thereof. Most preferably tartaric acid is used.

Suitable inorganic acids are phosphoric acid, boric acid and silicic acid. A preferred inorganic acid is phosphoric acid.

### Excipients

The following information on excipients is equally applicable to the first and third embodiment. As far as the second embodiment is concerned, the following information is applicable only to the extent that no excipients capable of accepting proton may be used.

The **diluent** can be selected from the group consisting of polysaccharides, mono- or disaccharides or sugar alcohols, preferably it is selected from starch, cellulose, sugar alcohols, or a mixture thereof. The polysaccharide is preferably a polysaccharide comprising from 200 to 10,000 monosaccharide residues, preferably 500 to 10,000 monosaccharide residues, preferably glucose residues. Cellulose may be selected from powdered cellulose, microcrystalline cellulose, silicified microcrystalline cellulose. Starch may be selected from starches from any suitable botanical source, for example corn, wheat, rice, tapioca, potato, preferably starches are modified starches, such as pregleatinized starch, which is a type of modified starch that has been processed to render the starch more flowable and directly compressible. Partially or wholly pregelatinized starches can be used. Monosaccharides, oligosaccharides and sugar alcohols may be selected from glucose, fructose, sucrose, lactose monohydrate, anhydrous lactose, raffinose, isomaltose, trehalose, dextrates, mannitol, erythritol, sorbitol, maltitol, xylitol and lactitol, compressible sugars, and mixtures thereof. Preferably the diluent is not selected from lactose, calcium salts of phosphoric acid or carbonic acid or sulfuric acid, such as calcium hydrogen phosphate anhydrous or hydrate or calcium carbonate. If such less preferred fillers are nevertheless employed, it is important to provide a sufficient amount of acid in order to accomplish a satisfactory level of stability.

In one aspect, low moisture diluents are advantageously used, having a loss on drying of less than 8% w/w, preferably less than 5% w/w more preferably less than 2.5% and most preferably less than 1.5% w/w, measured by halogen dryer, such as Mettler Toledo HR73 or R 83 or equivalent equipment. According to a preferred aspect, one or more of the further excipients of the formulation also fulfils the above condition of dryness. According to a particularly preferred aspect, all excipients fulfil this dryness condition.

According to a particularly preferred embodiment, the diluent is selected from cellulose powders (Ph. Eur.), microcrystalline celluloses and/or sugar alcohols. More preferably, the pharmaceutical formulation of the present invention comprises at least one diluent, selected from microcrystalline cellulose or mannitol.

Microcrystalline cellulose is preferably selected from microcrystalline cellulose with average particle size from 10 µm to 200 µm, preferably from 20 to 150 µm and/or moisture content ≤ 5%. Particularly, microcrystalline cellulose may be selected from microcrystalline cellulose with an average particle size 20 µm and a moisture content ≤ 5%, such as Avicel PH-105, microcrystalline cellulose with average particle size 50 µm and a moisture content ≤ 5%, such as Avicel PH-101 or Vivapur 101, microcrystalline cellulose with average particle size 50 µm and a moisture content ≤ 2%, such as Avicel PH-113, microcrystalline cellulose with average particle size 90 to 120 µm and a moisture content ≤ 5%, such as Avicel PH-102 or Vivapur 102, microcrystalline cellulose with average particle size 90 to 120 µm and a moisture content ≤ 1.5%, such as Avicel PH-112.

Mannitol may preferably be selected from mannitol with average particle size 50-300 µm, preferably from 100-250 µm.

It has been surprisingly found that the use of a mixture of microcrystalline cellulose and mannitol gives not only rise to formulations with physical and chemical stability, but that this combination of diluents allows to prepare such formulations with superior dose uniformity, weight variation and flow properties.

Preferably, the formulations of the present invention additionally comprise starch and more preferably modified starch (i.e. pregelatinised starch). Starch may be used as a binder, diluent, or disintegrating agent, whereas pregelatinised starch is used mainly as a binder and disintegrating agent. Modified starch may be selected from modified starch with an average particle size from 10 µm to 100 µm, preferably from 30 to 70 µm. Particularly, modified starch may be selected from modified starch with an average particle size of about 50 µm, such as Starch 1500 and Starch 1500 LM. The amount of starch, preferably modified starch, in the composition is typically in the range of about 1% to about 30% by weight. Preferably the amount of starch is about 5% to about 20%, more preferably about 10% to about 20%, by weight of the composition.

The amount of diluent in the composition is typically in the range of about 50% to about 99% by weight. Preferably the amount of the diluent is about 60% to about 95%, more preferably about 70% to about 90%, by weight of the composition.

The weight ratio of mannitol to microcrystalline cellulose in the composition is typically in the range of 5:1 to 1:5, preferably the weight ratio is in the range of 3:1 to 1:1, more preferably in the range of 2:1 to 1:1.

For the production of the formulations of the present invention it is preferred that the particle size of all excipients is of the same particle size range in order to minimize the segregation of the component of the mixture. The average particle sizes of the excipients used are preferably in the range of 20 to 200 µm, more preferably in the range of 50 to 150 µm. The main advantages of the use of the excipients with a particle size of the same degree are a high content uniformity due to a low segregation tendency, a good flowability providing a high weight consistency at various tableting/encapsulation speeds.

In the formulation of the invention the diluent may at the same time serve as a binder and/or disintegrant.

The pharmaceutical formulations of the present invention may contain additional ingredients selected from a wide variety of excipients known in the art of pharmaceutical formulation, such as disintegrants, binders, lubricants, and glidants.

Suitable **disintegrants** may be selected from crospovidone, starch, maize starch, pregelatinized starch, sodium starch glycollate, hydroxypropyl starch, microcrystalline cellulose, sodium and/or calcium salts of carboxymethyl cellulose, cross-linked carboxymethylcellulose (e.g. croscarmellose sodium and/or croscarmellose calcium), polacrilin potassium, low substituted hydroxypropylcellulose (L-HPC), sodium and/or calcium alginate, docusate sodium, methylcellulose, agar, guar gum, chitosan, alginic acid or mixtures thereof. According to one aspect of the invention, it is preferred to use a disintegrant other than sodium starch glycollate, sodium and/or calcium salts of carboxymethyl cellulose, cross-linked carboxymethylcellulose (e.g. croscarmellose sodium and/or croscarmellose calcium), polacrilin potassium, low substituted hydroxypropylcellulose (L-HPC), sodium and/or calcium alginate, docusate sodium Preferably, disintegrants are selected from maize starch, pregelatinized starch, crospovidone. Preferably the disintegrant is not selected from the group of compounds which may act as a proton acceptor, such as sodium and/or calcium salts of carboxymethyl cellulose, cross-linked carboxymethylcellulose (e.g. carboxymethylcellulose sodium, croscarmelose sodium), polacrilin potassium, sodium and/or calcium alginate, docusate sodium.

Suitable **binders** may be selected from povidone (polyvinylpyrrolidone), copovidone (vinylpyrrolidone-vinyl acetate copolymer), powdered cellulose, crystalline cellulose, microcrystalline cellulose, silicified microcrystalline cellulose, cellulose derivatives such as hydroxymethylcellulose, hydroxyethylcellulose, hydroxypropylcellulose, low substituted hydroxypropylcellulose and hydroxypropylmethylcellulose or other cellulose ethers, corn or potato or rice starch, pregelatinised starch, α-starch or dextrin, gum arabic, pullulan, polymethacrylates or mixture of binders. Preferably, the dry binders can be selected from copovidone, povidone, hydroxypropylcellulose, hydroxypropylmethylcellulose, microcrystalline cellulose, starch derivatives such as pregelatinised starch, low substituted hydroxypropylcelulose or mixtures thereof. More preferably, the pharmaceutical formulation of the present invention comprises at least one binder, selected from microcrystalline cellulose, hydroxypropylcellulose or starch or mixtures thereof. Microcrystalline cellulose and starches has simultaneously the role of a binder, a disintegrant and of a diluent and thus the use of an additional binder and/or disintegrant can be avoided if microcrystalline cellulose and/or starch is used in the formulation of the invention.

**Lubricants and glidants** are preferably used in the formulations of the invention in the usual standard way.

Suitable lubricants may be selected from lubricants, fatty acids, fatty acid esters, including glyceride esters (e.g., selected from glyceryl monostearate, glyceryl tribehenate, and glyceryl dibehenate), the group of metal salts of fatty acids with 12 to 20 carbon atoms such as magnesium, calcium, aluminum or zinc stearate, magnesium palmitate and magnesium oleate, hydrogenated vegetable oil, hydrogenated castor oil, talc, wax (e.g. STEROTEX NF, Lubriwax (Hydrogenated Vegetable Oil Type), meads wax or spermaceti), boric acid, sodium stearyl fumarate, macrogols, sugar esters (e.g., sorbitan monostearate and sucrose monopalmitate), inorganic materials (e.g., talc), polymers (e.g., PEG) or mixtures thereof. Advantageously, a lubricant other than metal salts of fatty acids with 12 to 20 carbon atoms such as magnesium, calcium, aluminum or zinc stearate, magnesium palmitate and magnesium oleate and other than sodium stearyl fumarate is selected. Preferably, lubricants are selected from stearic acid, glyceryl behenate, hydrogenated vegetable oil, or mixtures thereof; most preferably glyceryl behenate or stearic acid is used. Preferably the formulation is free of lubricants which may act as proton acceptor such as metal salts of fatty acids with 12 to 20 carbon atoms such as magnesium, calcium, aluminum or zinc stearate, magnesium palmitate and magnesium oleate, or sodium stearyl fumarate, talc.

The pharmaceutical formulation of the present invention can furthermore comprise one or more **glidants.** In some aspects, the one or more glidants are selected from colloidal silica, talc and magnesium trisilicate. In a preferred aspect, talc and magnesium silicate are not used. Most preferably anhydrous colloidal silica is used.

In preferred aspects of the invention, the active ingredient is evenly distributed in a matrix formed by the other ingredients of the formulation. According to a more preferred embodiment, the pharmaceutical formulation of the present invention comprises:
Lenalidomide (to be added as salt):0.2-20%, more preferably 1-10%, most preferably 3-8%;

| | |
|---|---|
| diluent: | 50-99%, more preferably 60-95%, most preferably 70-90%; |
| acid: | 0-10%, more preferably 2-8%, most preferably 3-7% |
| binder: | 0-30%, more preferably 0-20%, most preferably 0-15%; |
| disintegrant: | 0-30%, more preferably 0-20%, most preferably 0-15%; |
| lubricant: | 0.25-10%, more preferably 1-5%, most preferably 1.5-3%; |
| glidant: | 0.25-10%, more preferably 1-5%, most preferably 1.5-3%; |

wherein the pharmaceutical formulation preferably does not contain any excipients which are capable of acting as an acceptor of proton.

Lenalidomide is to be added in the form of a pharmaceutically acceptable salt thereof. However, the above relative amount indications are to be calculated on the basis of the content of the lenalidomide free base. In those aspects, wherein the pharmaceutical formulations of the present invention comprise of an excipient which has multiple functions, i.e. one excipient is used as diluent and/or binder etc., the permissible relative amount of the excipient is defined by the higher percentage specified in the above formulation. For example, when the formulation of the present invention comprises microcrystalline cellulose, which can act as diluent, binder and disintegrant, its ratio is defined by the highest ratio indication as defined, i.e. 50-99% of diluent. Each function of the excipient with multiple functions cannot be defined separately.

In a preferred aspect, the formulation of the invention comprises 1 to 10% of lenalidomide (in the form of a pharmaceutically acceptable salt, preferably lenalidomide hydrochloride), 60 to 95 % by weight of a diluent selected from microcrystalline cellulose and/or mannitol, 3-8% by weight of an acid, 0 to 15 % by weight of a binder selected from pregelatinized starch or hydroxypropylcellulose and 0 to 5 % by weight of an disintegrant preferably selected from low substituted hydroxypropylcellulose (L-HPC), and 0.25 to 5 % by weight of a lubricant, preferably selected from glyceryl behenate or stearic acid, and 0.25 to 5 % by weight of a glidant, preferably selected from colloidal anhydrous silica .

In an even more preferred aspect, the formulation of the invention comprises from 1 to 10% of lenalidomide (in the form of a pharmaceutically acceptable salt, preferably lenalidomide hydrochloride), from 60 to 95 % by weight of a diluent selected from microcrystalline cellulose and mannitol and combinations thereof, 3-8% of tartaric acid, 5 to 15 % by weight of a binder preferably selected from pregelatinized starch, and 1 to 5 % by weight of a lubricant, preferably selected from glyceryl behenate.

The invention also relates to a packaged pharmaceutical composition comprising the pharmaceutical composition described above, which is present in a low gas and moisture permeable primary packaging. The low gas and moisture permeable primary packaging may comprise materials such as aluminium or polychloro-3-fluoroethylene homopolymer/PVC laminate. Typically, the thickness of the packaging will be in the range of 10 to 40 µm for AI/AI blisters and 10 to 110 µm for Alpolychloro-3-fluoroethylene homopolymer/PVC laminate blisters. Optionally, the packaged pharmaceutical composition may further comprise a desiccant. The desiccant may be placed inside the packaging unit together with a dosage form such as a tablet and/or in the closure system and/or can be incorporated into the walls of the primary packaging unit. For example, the pharmaceutical composition can be packaged in containers made of glass or polymers, with or without desiccant.

### Method of the present Invention

The pharmaceutical formulations of the present invention may be manufactured using wet or dry methods. The present invention provides, as a further embodiment, methods for preparing the formulations of the present invention. The methods described below are suitable for the preparation of all pharmaceutical formulations of the present invention (to the extent that the components of the individual pharmaceutical formulations are compatible with the components mentioned in connection with the methods described below).

According to one aspect, the pharmaceutical formulations are prepared by wet methods such as wet granulation.

According to a preferred aspect of this embodiment of the present invention, the pharmaceutical formulations are prepared by dry methods such as direct compression, dry blending, or dry granulation, and more preferably by dry blending.

The present inventors have found that the formulations prepared by dry processes exhibit improved dissolution profile, physical and chemical stability compared to the formulations prepared by wet granulation.

It was surprisingly found by the present inventors that stable pharmaceutical formulations comprising lenalidomide in the form of pharmaceutically acceptable salt as the active ingredient, which exhibits high storage stability and excellent dose uniformity, can be prepared by a simple dry blending process if lenalidomide in the form of pharmaceutically acceptable salt is first homogeneously mixed with certain excipient and then subjected to sieving, followed by optional addition of further excipients, optional mixing, optional sieving and compressing or encapsulating the mixture. The blending of the compounds may be achieved in one or more steps. For instance, the active ingredient may first be mixed with a part of the diluent and this mixture can then be combined with a mixture of other ingredients. The whole process is preferably performed in the absence of a solvent.

The most significant advantages concerning the formulation quality are the processing without any need for moisture and heat that are inherent in most wet granulation procedures. Such a process can improve the stability of the formulations of the present invention.

Among the preferred dry methods, dry blending is most preferred as this allows to avoid high compaction pressures involved in slugging or roller compaction, so that a further stabilization effect can be accomplished by means of dry blending.

The methods described below are intended for preparing the pharmaceutical formulations of the present invention. This means that the ingredients (API, excipients) mentioned below must satisfy the essential features described for the pharmaceutical formulations of the present invention and that they preferably satisfy the features described as preferred features of the pharmaceutical formulations of the present invention.

In a preferred aspect of this embodiment of the present invention, a process for preparing the pharmaceutical formulations of the present invention is provided, which comprises:
- combining a lenalidomide pharmaceutically acceptable salt and at least one excipient or a mixture of excipients;
- optionally sieving;
- mixing to give a mixture
- compressing the mixture to desired solid dosage form or encapsulating the mixture.

In a more preferred aspect of this embodiment of the present invention, the process for preparing the pharmaceutical formulations of the present invention comprises:
- mixing lenalidomide pharmaceutically acceptable salt and a portion of diluent and/or further excipients;
- sieving the mixture;
- mixing the mixture with the remaining portion of diluent and/or further excipients in a mixer to obtain a homogenized mixture,
- optionally dry sieving the mixture through a sieve,
- addition of lubricant and/or glidant and mixing to obtain a compression mixture,
- compressing the compression mixture to the desired solid dosage form or encapsulating the compression mixture.

The above-mentioned dry sieving step may be advantageous in order to segregate cohesive particles and to improve content uniformity.

In an even more preferred aspect of this embodiment of the present invention, a process for preparing the pharmaceutical formulations of the present invention is provided, which comprises:
- mixing lenalidomide pharmaceutically acceptable salt and a portion of diluent in a weight/weight ratio from 1:1 to 1: 50, preferably from 1:2 to 1:30, more preferably from 1:3 to 1:25, and/or further excipients,
- sieving the mixture,
- mixing the above obtained mixture with the remaining portion of diluent and/or further excipients in a mixer to obtain a homogenized mixture,
- optionally dry sieving the mixture through a sieve,
- addition of lubricant and/or glidant and mixing to obtain a mixture,
- compressing the mixture to desired solid dosage form or encapsulating the mixture.

The above-mentioned dry sieving step may be advantageous in order to segregate cohesive particles and to improve content uniformity.

Alternatively the composition of the present invention can be prepared by dry granulation processes.

The dry granulation process comprises a process which comprises:
- optionally sieving lenalidomide pharmaceutically acceptable salt and at least one excipient or a mixture of excipients,
- granulating lenalidomide pharmaceutically acceptable salt and at least one excipient or a mixture of excipients using processes known in the art as slugging and/or roller compaction;
- optionally milling and sieving the obtained slugs or compacts to obtain a granulate with desired particle size distribution;
- addition of one or more excipients or a mixture of excipients to the granulate followed by mixing to give a mixture;
- compressing the mixture to the desired solid dosage form or encapsulating the mixture;
or a process which comprises:
- optionally sieving lenalidomide pharmaceutically acceptable salt and at least one excipient or a mixture of excipients;
- granulating a portion of optionally sieved lenalidomide pharmaceutically acceptable salt and at least one excipient or a mixture of excipients using processes known in the art as slugging and/or roller compaction;
- optionally milling and sieving the obtained slugs or compacts to obtain a granulate with the desired particle size distribution;
- addition of the rest of the lenalidomide pharmaceutically acceptable salt and at least one excipient or a mixture of excipients to the granulate to give a mixture;
- compressing the mixture to the desired solid dosage form or encapsulating the mixture.

The mixing of lenalidomide pharmaceutically acceptable salt and at least one excipient or a mixture of excipients may be effected in conventional devices used for mixing of powder, e.g. motionless (passive) mixers, fluidized bed, diffusion, biconic diffusion, biconic, tubular, cubic, planetary, Y-, V-shaped or high-shear mixers.

In another embodiment of the present invention, the same equipment may be used in the preparation of a compression mixture with the prior step of a granulate preparation by a granulation as described by the terms "dry granulation".

Dry granulation can be performed by processes known in the art as slugging and/or roller compaction, the latter being preferred. Lenalidomide pharmaceutically acceptable salt, optionally sieved to eliminate agglomerates, is usually mixed and dry-granulated with at least one excipient or a mixture of excipients into slugs/compacted material by using roller compactor or compression machine. The obtained slugs/compacted material is crushed and optionally sieved to obtain a uniform distribution of the granules of a dry granulate.

Alternatively the composition of the present invention can be prepared by wet granulation processes.

The wet granulation process comprises a process which comprises:
- dissolving or suspending an acid and optionally at least one excipient in a solvent selected from the group of water, alcohols, such as ethanol, propanol, 2-propanol, butanol, ketones, such as acetone, methyl ethyl ketone, tert-butyl methyl ketone, or a mixture thereof, the solvent being preferably selected from water, to give a granulation liquid;
- granulating a mixture of optionally sieved lenalidomide pharmaceutically acceptable salt and at least one excipient or a mixture of excipients with granulation liquid using processes known in the art such as fluid bed granulator or high shear granulator
- optionally drying, milling and/or sieving the obtained granulate to obtain a granulate with the desired particle size distribution;
- addition of the rest of at least one excipient or a mixture of excipients to the granulate to give a mixture;
- compressing the mixture to the desired solid dosage form or encapsulating the mixture.
or a process which comprises:
- dissolving or suspending an acid in a solvent selected from the group of water, alcohols, such as ethanol, propanol, 2-propanol, butanol, ketones, such as acetone, methyl ethyl ketone, tert-butyl methyl ketone, or a mixture thereof, the solvent being preferably selected from water, to give a granulation liqiuid;
- mixing excipients with the exception of lubricant to give a mixture of excipients
- granulating a mixture of excipients with granulation liquid using processes known in the art such as fluid bed granulator or high shear granulator
- optionally drying, milling and/or sieving the obtained granulate to obtain a granulate with the desired particle size distribution;
- addition of lenalidomide pharmaceutically acceptable salt and of the rest of at least one excipient or a mixture of excipients, including lubricant, to the granulate to give a mixture;
- compressing the mixture to the desired solid dosage form or encapsulating the mixture.

The following examples illustrate the invention and are not intended to restrict the scope of the invention, as defined by the appended claims, in any way.

### Examples

### Methods

### Stability testing

The binary mixtures and pharmaceutical compositions were stored in vials at 40°C/75%RH for 1 month or at 50° C 75% RH for 14 days. Disproportionation effect was monitored by X-ray powder diffraction pattern. XPRD data was obtained in order to determine if a sample remains in the starting acid addition salt form, indicating that it is stable under these conditions or transformed partially or completely into a different crystalline material such as free base form, indicating that it is not stable under these conditions.

**X-ray powder diffraction patterns** were obtained by a PANalytical PW3040/60 X'Pert PRO diffractometer using CuKα radiation of 1,541874.

### pH value of pharmaceutical formulation:

The pH of a pharmaceutical formulation (20% (w/v) suspension) was determined as follows: Tablets were finely powdered in a mortar or a capsule shell was opened and the powder was optionally finely powdered in mortar and a 20% (w/v) suspension of the powder was prepared in freshly boiled and cooled water. After stirring the suspension for 15 minutes the pH value of the sample suspension was measured at a temperature of between 20°C and 25°C on a suitable, calibrated pH meter Radiometer PHM240 using combined glass electrode XC161 .

### Examples

**Binary mixtures** of excipients as disclosed in Table 1 were prepared and subjected to stress stability testing.

**Table 1**

| | **API** | **Ref. Ex 1** | **Ref. Ex 2** | **Ref. Ex 3** | **Ref. Ex 4** |
|---|---|---|---|---|---|
| Lenalidomid HCl·H2O | + | + | + | + | + |
| Microcrystalli ne cellulose | | | | | |
| mannitol | | | + | + | + |
| isomalt | | + | | | |
| Mg stearate | | | + | + | + |
| Sodium croscarmello se | | | | + | |
| Starch 1500 | | | | | + |
| Stability testing 1 month at 40°C/75%RH | HCl salt | HCl salt | Mixture of HCl salt and free base | Mixture of HCl salt and free base | Mixture of HCl salt and free base |

### Examples according to invention and reference examples for preparing pharmaceutical composition are disclosed in Table 2, Table 3 and Table 4

**Table 2**

| | **Ref. Example 5** | **Example 1** | **Example 2** | **Example 3** | **Example 4** | **Example 5** |
|---|---|---|---|---|---|---|
| Lenalidomid HCl·H₂O | 30 mg | 30 mg | 30 mg | 30 mg | 30 mg | 30 mg |
| Microcrystalline cellulose MCC 102 | 150 mg | 150 mg | 150 mg | 150 mg | 150 mg | 150 mg |
| Mannitol | 200 mg | 200 mg | 200 mg | 200 mg | 200 mg | 200 mg |
| Tartaric acid | | 15 mg | | | 15 mg | |
| Citric acid | | | 20 mg | | | 20mg |
| Mg stearate | 4 mg | | | | | |
| Sodium croscarmellose | 12 mg | | | | | |
| Starch 1500 | | | | 40 mg | 40mg | 40mg |
| Stability testing 1month at 40°C/75%RH | Mixture of HCl salt and free base | HCl salt | HCl salt | HCl salt | HCl salt | HCl salt |
| Stability testing 3 month at 30°C/65%RH | Mixture of HCl salt and free base | HCl salt | HCl salt | HCl salt | HCl salt | HCl salt |

**Table 3**

| | **Example 6** | **Example 7** | **Example 8** | **Example 9** | **Example 10** | **Example 11** | **Example 12** |
|---|---|---|---|---|---|---|---|
| Lenalidomid HCl·H₂O | 30 mg | 30 mg | 30 mg | 30 mg | 30 mg | 30 mg | 30 mg |
| MCC 102 | 150 mg | 150 mg | 150 mg | 150 mg | 150 mg | 150 mg | 150 mg |
| Mannitol | 200 mg | 200 mg | 200 mg | 200 mg | 200 mg | 200 mg | 200 mg |
| Tartaric acid | 15 mg | | | 15 mg | | 15 mg | |
| Citric acid | | 20 mg | | | 20 mg | 20mg | 20mg |
| L-HPC | 40mg | 40mg | | | | | |
| Stearic acid | | | 8 mg | 8 mg | 8 mg | | |
| Glyceryl behenate | | | | | 8 mg | 8 mg | 8 mg |
| Stability testing 1 month at 40°C/75%RH | HCl salt | HCl salt | HCl salt | HCl salt | HCl salt | HCl salt | HCl salt + |
| Stability testing 3 month at 30°C/65%RH | HCl salt | HCl salt | HCl salt | HCl salt | HCl salt | HCl salt | HCl salt |

### Process for preparation of pharmaceutical compositions of Examples and Reference Examples

Lenalidomide hydrochloride and the all the excipients with respect to the composition were weighed into the glass vial. Gelatine capsule were added into the vials. The vials were then closed with the stopper and gently mixed to form a homogenous mixture. The vials were subjected to the stress conditions.

### Reference examples according to WO2011050962

The pharmaceutical compositions were prepared in accordance with the disclosure in WO2011050962 (Example 1 and Example 3, binary mixtures were prepared with dicalcium phosphate dihydrate which was according to WO2011050962, p. 23, I. 6-23, taught as excipient providing enhanced storage stability)

**Table 4**

| | **Ref. Ex. 6** | **Ref. Ex. 6** | **Ref. Ex. 7** |
|---|---|---|---|
| Lenalidomid HCl·H₂O | 22.8 mg | 17.1 mg | 17.1 mg |
| MCC 102 | 160 mg | 120 mg | |
| Lactose anhydrous | 240 mg | | |
| Dicalcium hydrogen phosphate | / | 381 mg | 381 mg |
| Tartaric acid | | | |
| Ac-Di-Sol | 24 mg | 18 mg | |
| Mg stearate | 8 mg | 6 mg | |
| Stability testing 1 month at 40°C/75%RH | Free base | Free base | Free base |

### Process for preparation of pharmaceutical compositions according to Reference Examples

The mixtures were prepared as physical mixtures in mass ratio according to the composition disclosed in WO2011050962.

Lenalidomide hydrochloride in the form of monohydrate form VII and the all the excipients with respect to the composition were weighed into the glass vial. Gelatine capsule were added into the vials. Vials were then closed with the stopper and gently mixed to form a homogenous mixture. The vials were subjected to the stress conditions.

### Stability testing

As evident from the results of stability testing, excipients capable of accepting protons, such as magnesium stearate, sodium croscarmellose, or dicalcium hydrogen phosphate induced conversion of the salt into free base form of lenalidomide. On the other hand, the presence of an additional acid and/or the absence of basic excipients stabilized the physical form lenalidomide acid addition and disproportionation is not observed at all or was observed in only minor amounts.

### Examples according to the present invention

### Capsule formulation

**Table 5**

| | **Example 13** | **Example 14** | **Example 15** | **Example 16** | **Example 17** | **Example 18** | **Example 19** |
|---|---|---|---|---|---|---|---|
| | mg/capsule | | | | | | |
| Lenalidomid HCl·H₂O | 30.25 | 30.25 | 30.25 | 30.25 | 30.25 | 30.25 | 30.25 |
| Microcrystalline cellulose Avicel PH 102 | 146.75 | 106.75 | 106.75 | 114.25 | 91.75 | 102.75 | 102.75 |
| Mannitol (Parteck M200) | 200.00 | 200.00 | 200.00 | 200.00 | 200.00 | 200.00 | 200.00 |
| Tartaric acid | 15.00 | 15.00 | 15.00 | 7.50 | 30.00 | 15.00 | 15.00 |
| L-HPC LH21 | | | 40.00 | 40.00 | 40.00 | 40.00 | 40.00 |
| Starch 1500 | - | 40.00 | - | - | - | - | - |
| Glyceryl behenate Compritol 888 | 8.00 | 8.00 | 8.00 | 8.00 | 8.00 | 8.00 | 8.00 |
| HPC Klucel EF | - | - | - | - | - | - | 4.00 |
| Total | 400.00 | 400.00 | 400.00 | 400.00 | 400.00 | 400.00 | 400.00 |
| Stability testing 40°C/75%RH 1 month | HCl salt | HCl salt | HCl salt | HCl salt | HCl salt | HCl salt | HCl salt |

### Process for Examples 12 to 16

Lenalidomide hydrochloride and a half part of mannitol were mixed with turbula and sieved through a 0.50 mm sieve to obtain a triturate mixture. The mixture was than blended with other parts of disintegrant (Starch or L-HPC), tartaric acid, and the remaining part of mannitol and sieved through a 0.50 mm sieve to obtain a homogenized mixture. Avicel was used for rising of the mesh and was added to the mixture. the blend was mixed and Compritol 888 was added and blended. The composition was encapsulated into capsules comprising 30.25 mg of lenalidomide hydrochloride (equivalent to 25 mg of lenalidomide base base)

### Process for Examples 17 and 18:

A granulation solution was prepared by dissolving Klucel and tartaric acid in water. A mixture of lenalidomide hydrochloride, Parteck, Avicel, and a half part of L-HPC LH21 was prepared in a mortar. The mixture was granulating with a granulating solution to obtain the granules, an additional part of water was added and further granulated. The granulate was dried in an oven. After sieving the granulate through a 1.0 mm sieve, the granules were finally blended with the remaining part of L-HPC LH21. To the blend sieved Compritol 888 was added and mixed. The composition was encapsulated into capsules comprising 30.25 mg of lenalidomide hydrochloride (equivalent to 25 mg of lenalidomide free base).

Characteristics of lenalidomide hydrochloride monohydrate particles used in presented formulations

**Table 6**

| **Average diameter (µm)** | **d(0.1) (µm)** | **d(0.5) (µm)** | **d(0.9) (µm)** |
|---|---|---|---|
| 10,6 | 4,0 | 8,8 | 21 |

### Stability testing

The capsules were stored in vials at 40°C/75%RH for 1 month. Physical stability of the samples was monitored by X-ray powder diffraction pattern. As evident from the results, the physical form remains the same, a disproportionation effect was not observed.

### Additional examples according to invention

### Capsule formulation

**Table 7**

| | **Example 20** | **Example 21** | **Example 22** | **Example 23** | **Example 24** | **Example 25** | **Example 26** | **Example 27** |
|---|---|---|---|---|---|---|---|---|
| | mg/ capsule | mg/ capsule | mg/ capsule | mg/ capsule | mg/ capsule | mg/ capsule | mg/ capsule | mg/ capsule |
| Lenalidomide HCl·H₂O | 30.25 | 30.25 | 30.25 | 30.25 | 30.25 | 30.25 | 30.25 | 6.05 |
| Mannitol M200 | 200.00 | 200.00 | 200.00 | 200.00 | 200.00 | - | - | 100.00 |
| Mannitol M100 | - | - | - | - | - | 200.00 | 106.40 | - |
| MCC PH102 | 106.40 | 91.05 | 109.88 | 112.54 | 106.40 | - | - | 62.27 |
| MCC PH101 | - | - | - | - | - | 106.40 | 200.00 | - |
| Starch 1500 | 40.00 | 40.00 | 40.00 | 40.00 | 40.00 | 40.00 | 40.00 | 20.00 |
| Compritol 888 | 8.00 | 8.00 | 8.00 | 8.00 | - | 8.00 | 8.00 | 4.00 |
| Stearic acid | - | - | - | - | 8.00 | - | - | - |
| Tartaric acid | - | 30.70 | - | - | 15.35 | 15.35 | 15.35 | 7.68 |
| Milled tartaric acid | 15.35 | - | - | - | - | - | - | - |
| Maleic acid | - | - | 11.87 | - | - | - | - | - |
| Oxalic acid | - | - | - | 9.21 | - | - | - | - |
| Total | 400.00 | 400.00 | 400.00 | 400.00 | 400.00 | 400.00 | 400.00 | 200.00 |

### Process for preparation of pharmaceutical compositions according to additional examples 20 to 27

Triturate was prepared with API, acid (tartaric, maleic or oxalic) and MCC (triturate 1). The obtained triturate was mixed and sieved on a mesh with size 0.5 mm. After that, disintegrant (Starch 1500) and 1/2 of the mannitol were added. The thus obtained blend was mixed and sieved on a mesh with size 0.5 mm (triturate 2). The rest of the mannitol was used for rinsing of the mesh and then added to the triturate 2. The blend was mixed long enough to obtain suitable homogeneity of the API. At the end, sieved lubricant (Compritol 888 or stearic acid; sieved by using a 0.5 mm sieve) was added and the obtained blend was mixed. Capsules were filled up by manual filling using tamping as an intermediate step.

### Examples of formulations with acid coated excipients

**Table 8**

| | **Example 28** | **Example 29** | **Example 30** |
|---|---|---|---|
| | mg/capsule | mg/capsule | mg/capsule |
| Lenalidomide HCl·H₂O | 30 | 30 | 30 |
| Mannitol M200 | 200 | 200 | 200 |
| MCC PH102 | 107 | 107 | 107 |
| Starch 1500 | 40 | 40 | 40 |
| Tartaric acid | 15 | - | - |
| Maleic acid | - | 15 | - |
| Oxalic acid | - | - | 15 |
| Compritol 888 | 8 | 8 | 8 |
| Total | 400 | 400 | 400 |

### Process for preparation of examples 28 to 30

The prescribed amount of acid (tartaric, maleic or oxalic) was dissolved in 20 g of water. Fluidized bed process was used to slowly spray the solution on excipients (Mannitol, MCC and Starch) and prevent particles agglomeration. After that, sieved lubricant (Compritol 888, sieved by using a 0.5 mm sieve) was added and the obtained blend was mixed. At the end, the API was added and the obtained blend was mixed long enough to obtain a suitable homogeneity of the API. Capsules were filled up with manual filling using tamping as intermediate step.

### Example of wet granulation formulation

**Table 9**

| | **Example 31** |
|---|---|
| | mg/capsule |
| Lenalidomide HCl·H₂O | 30,25 |
| Mannitol | 200,00 |
| MCC | 102,75 |
| Tartaric acid | 15,00 |
| L-HPC LH21 | 40,00 |
| Compritol 888 | 8,00 |
| Klucel EF | 4,00 |
| Total | 400,00 |

### Process for preparation of Example 31: wet granulation formulation

A fluid bed process was used to perform wet granulation. Klucel EF was dissolved in a sufficient amount of solvent (purified water, ethanol, isopropanol, acetone or a mixture of any of the mentioned solvents with water) to obtain a 4 % solution. After that, the whole amount of tartaric acid was added to the solution. Diluents, API and 1/2 of disintegrant were mixed in a fluid bed granulator to obtain suitable homogeneity of the API. Granulation liquid was sprayed on the blend to achieve adequate agglomeration of the powder blend. The spraying system was rinsed with purified water once the spraying of binder solution was finished. The thus obtained granulate was dried and sieved through 1 mm mesh. Sieved 1/2 of disintegrant was added to the granulate and the obtained blend was mixed. At the end, sieved Compritol was added to granulate and the obtained blend was mixed again. Capsules were filled up with manual filling using tamping as intermediate step.

## Claims

1. A pharmaceutical formulation, which is a solid formulation selected from the following embodiments (A), (B) and (C):
(A) a pharmaceutical formulation comprising (a) lenalidomide in the form of a pharmaceutically acceptable acid addition salt, (b) an acid and (c) at least one further pharmaceutically acceptable excipient;
(B) a pharmaceutical formulation, comprising (a) lenalidomide in the form of a pharmaceutically acceptable acid addition salt, (b) optionally an acid, (c) at least one further pharmaceutically acceptable excipient and (d) wherein it is free of excipients capable of accepting proton; and
(C) a pharmaceutical formulation, comprising (a) as the active ingredient, lenalidomide in the form of hydrochloride acid addition salt, (c) one or more pharmaceutically acceptable excipients, wherein (e) the excipients are selected such that the pharmaceutical composition shows acidic properties.

2. The pharmaceutical formulation according to Claim 1, embodiments (A) or (C), which comprises (b) an acid.

3. The pharmaceutical formulation according to Claim 1, embodiments (B) or (C), which (d) is free of excipients capable of accepting proton.

4. The pharmaceutical formulation according to Claim 1, embodiment (C), which comprises an acid and is free of excipients capable of accepting proton.

5. The pharmaceutical formulation according to any one of Claims 1 to 4, which comprises lenalidomide in the form of hydrochloric acid addition salt.

6. The pharmaceutical formulation according to any one of Claims 1 to 5, which comprises at least one diluent, selected from microcrystalline cellulose and mannitol, preferably a combination of microcrystalline cellulose and mannitol.

7. The pharmaceutical formulation according to any one of Claims 1 to 6, which comprises an acid that has a pKa of 4 or less, preferably in the range of 1 to 3.

8. The pharmaceutical formulation according to any one of Claims 1 to 7, wherein the diluent has a loss on drying of less than 5% w/w, preferably less than 2% w/w and most preferably less than 1.5% w/w.

9. The pharmaceutical formulation according to Claim 8, wherein all excipients have a loss on drying of less than 5% w/w, preferably less than 2% w/w and most preferably less than 1.5% w/w.

10. The pharmaceutical formulation according to any one of Claims 1 to 9, wherein the formulation has a pH of 1 to 3, preferably 1 to 2.5, when measured as a 20 % w/v suspension in water at 25°C.

11. A process for manufacturing the pharmaceutical formulation according to any one of Claims 1 to 10, which comprises the steps of
- combining lenalidomide in the form of a pharmaceutically acceptable acid addition salt, preferably the hydrochloride salt, and one or more excipients;
- mixing to give a mixture and
- compressing the mixture or filling the mixture into a capsule shell.

12. The process according to Claim 11, which is carried out in the absence of a solvent.

13. The process according to Claim 11 or 12, which further comprises a step of sieving, which is carried out before and/or after the step of mixing.

14. The process according to any one of Claims 11 to 13, which further comprises a step of addition of lubricant and/or glidant after optional sieving and/or after mixing.

15. The process according to any one of Claims 11 to 14, which comprises the following steps:
- mixing lenalidomide pharmaceutically acceptable salt, preferably hydrochloride, and a portion of a diluent in a weight/weight ratio from 1:1 to 1:50, preferably from 1:2 to 1:30, more preferably from 1:3 to 1:25, and/or further excipients and sieving the mixture;
- mixing the above obtained mixture with the remaining portion of diluent and/or further excipients in a mixer to obtain a second mixture,
- optionally dry sieving the mixture through a sieve,
- addition of lubricant and/or glidant followed by mixing to obtain a third mixture,
- compressing the third mixture or filling the mixture into a capsule shell.
